# EUROPEAN PATENT SPECIFICATION

(11) **EP 3 882 340 B1**
(45) Date of publication and mention of the grant of the patent: **15.01.2025**
(21) Application number: 19885990.2
(22) Date of filing: 11.11.2019
(51) Int. Cl.: C12N 5/071, C12N 5/077, C12N 5/0775, A61K 35/28, A61K 35/33, A61K 35/12

(54) **METHOD FOR PRODUCING INSULIN-PRODUCING CELLS, AND COMPOSITION**
VERFAHREN ZUR HERSTELLUNG VON INSULINPRODUZIERENDEN ZELLEN UND ZUSAMMENSETZUNG
PROCÉDÉ DE PRODUCTION DE CELLULES PRODUISANT DE L'INSULINE, ET COMPOSITION

(30) Priority: 14.11.2018 JP 2018213449
(43) Date of publication of application: 22.09.2021
(73) Proprietor: Kataoka Corporation, Kyoto-shi, Kyoto 601-8203 (JP); Kyoto Prefectural Public University Corporation, Kyoto-shi, Kyoto 602-8566 (JP)
(72) Inventor: DAI, Ping, Kyoto-shi, Kyoto 602-8566 (JP); TAKEDA, Yukimasa, Kyoto-shi, Kyoto 602-8566 (JP); HARADA, Yoshinori, Kyoto-shi, Kyoto 602-8566 (JP); MATSUMOTO, Junichi, Kyoto-shi, Kyoto 601-8203 (JP); KUSAKA, Ayumi, Kyoto-shi, Kyoto 601-8203 (JP)
(74) Representative: Vossius & Partner Patentanwälte Rechtsanwälte mbB
(86) International application number: PCT/JP2019/044057
(87) International publication number: WO 2020/100788

(56) References cited:
- EP-A1- 3 031 905
- WO-A1-2010/055616
- WO-A1-2014/001771
- BHAWNA CHANDRAVANSHI ET AL: "Reprogramming mouse embryo fibroblasts to functional islets without genetic manipulation", JOURNAL OF CELLULAR PHYSIOLOGY, WILEY SUBSCRIPTION SERVICES, INC, US, vol. 233, no. 2, 11 August 2017 (2017-08-11), pages 1627 - 1637, XP071323042, ISSN: 0021-9541, DOI: 10.1002/JCP.26068
- D. FOMINA-YADLIN ET AL: "Small-molecule inducers of insulin expression in pancreatic -cells", PROCEEDINGS OF THE NATIONAL ACADEMY OF SCIENCES, vol. 107, no. 34, 9 August 2010 (2010-08-09), pages 15099 - 15104, XP055200934, ISSN: 0027-8424, DOI: 10.1073/pnas.1010018107
- FOMINA-YADLIN DINA A: ""Small molecule inducers of insulin expression in pancreatic alpha cells"", PHD DISSERTATION, 6 July 2011 (2011-07-06), pages 1 - 210, XP055848413, Retrieved from the Internet <URL:https://hollis.harvard.edu/primo-explore/fulldisplay?docid=01HVD_ALMA212155280430003941&context=L&vid=HVD2&lang=en_US&search_scope=everything&adaptor=Local%20Search%20Engine&tab=everything&query=any,contains,Fomina-Yadlin%20Dina&offset=0> [retrieved on 20211006]
- SAIYONG ZHU ET AL: "Human pancreatic beta-like cells converted from fibroblasts", NATURE COMMUNICATIONS, vol. 7, 6 January 2016 (2016-01-06), pages 10080, XP055430582, DOI: 10.1038/ncomms10080
- BHAWNA CHANDRAVANSHI, RAMESH BHONDE: "Reprogramming mouse embryo fibroblasts to functional islets without genetic manipulation", JOURNAL OF CELLULAR PHYSIOLOGY, vol. 233, no. 2, 28 June 2017 (2017-06-28), pages 1627 - 1637, XP055819513, ISSN: 0021-9541, DOI: 10.1002/jcp.26068
- ANANDWARDHAN A. HARDIKAR, BERNICE MARCUS-SAMUELS, ELIZABETH GERAS-RAAKA, BRUCE M. RAAKA, MARVIN C. GERSHENGORN: "Human pancreatic precursor cells secrete FGF2 to stimulate clustering into hormone-expressing islet-like cell aggregates", PROCEEDINGS OF THE NATIONAL ACADEMY OF SCIENCE, vol. 100, no. 12, 10 June 2003 (2003-06-10), pages 7117 - 7122, XP055819515, ISSN: 0027-8424, DOI: 10.1073/pnas.1232230100
- AMIT CHOUDHARY, KAIHUI HU HE, PHILIPP MERTINS, NAMRATA D. UDESHI, VLADO DANČÍK, DINA FOMINA-YADLIN, STEFAN KUBICEK, PAUL A. CLEMON: "Quantitative-Proteomic Comparison of Alpha and Beta Cells to Uncover Novel Targets for Lineage Reprogramming", PLOS ONE, vol. 9, no. 4, e95194, 23 April 2014 (2014-04-23), pages 1 - 10, XP055707997, DOI: 10.1371/journal.pone.0095194
- TAIOKA TOSHIHIRO: "Molecular mechanisms of insulin resistance in beta cells.", THE SHOWA UNIVERSITY JOURNAL OF MEDICAL SCIENCES, vol. 2, no. 2, 2011, pages 127 - 138, XP009528083, ISSN: 1884-7854
- XIAOJIE MA, SAIYONG ZHU: "Chemical strategies for pancreatic beta cell differentiation, reprogramming, and regeneration", ACTA BIOCHIMICA BIOPHYSICA SINICA, vol. 49, no. 4, 1 April 2017 (2017-04-01), pages 289 - 301, XP055647725, ISSN: 1672-9145, DOI: 10.1093/abbs/gmx008

## Description

### TECHNICAL FIELD

### (Cross-Reference to Related Applications)

This application claims the benefit of Japanese Patent Application No. 2018-213449, filed on November 14, 2018, with the Japanese Patent Office.

The present invention is classified to the technical field of regenerative medicine. The present invention relates in the technical field to an in vitro process for directly producing insulin-producing cells from somatic cells by low molecular weight compounds, and to low molecular weight compound-induced insulin-producing cells (ciIPCs: chemical compound-induced insulin-producing cells) produced by such a process. The present invention further relates to a differentiation induction medium composition which can be used for the process of producing the insulin-producing cells.

### BACKGROUND OF THE INVENTION

Nowadays, processes (direct reprogramming) for converting somatic cells such as fibroblasts, directly into other cells by low molecular weight compounds without gene transfer, are actively studied. For example, in Patent Document 1, several low molecular weight compounds such as an ALK5 inhibitor and an ALK6 inhibitor are combined, and somatic cells, in particular human fibroblasts, are cultured under their presence to directly induce brown adipocytes, osteoblasts, cartilage cells, nervous system cells, or cardiomyocytes from the somatic cells. As in the invention of Patent Document 1, it is very beneficial that conventional fibroblasts can be directly induced into cells such as brown adipocytes by a low molecular weight compound which is easy to obtain. For example, autologous fibroblasts can be conveniently used to produce other autologous cells, thereby increasing their applications in regenerative medicine. Moreover, cells can be easily produced as experimental materials for the development of new pharmaceuticals.

For insulin-secreting pancreatic β-cells, in addition to the reports of differentiation induction from iPS-cells or ES-cells to human pancreatic β-cells, it has been reported that endodermal cells such as pancreatic α-cells, pancreatic acinar cells, pancreatic ductal glandular cells, small intestinal crypt cells, hepatocytes, and cholangiocytes were directly induced to pancreatic β-cells using Pdx1, Ngn3, and MafA, which are pancreatic β-cell-specific transcription factors (Non-Patent Document 1). Moreover, direct induction from mouse embryonic fibroblasts (MEFs) or human dermal fibroblasts to pancreatic β-cells using Yamanaka-4-factors has been reported (Non-Patent Documents 2 and 3). Furthermore, it has been reported that endoderm progenitor cells were produced from mouse embryonic fibroblasts (MEFs) by low molecular weight compounds and differentiated into pancreatic endocrine cells (Non-Patent Document 4).

The differentiation induction from somatic cells to pancreatic β-cells and the like as described above is always performed by culturing in a medium containing various other compounds, nutrients and the like. The medium can include, for example, common media such as MEM (Minimum Essential Medium), DMEM (Dulbecco's Modified Eagle's Medium), and DMEM/F 12, or a modified medium thereof, and these are commercially available. Furthermore, in general, appropriate components (serum, proteins, amino acids, saccharides, vitamins, fatty acids, antibiotics, and the like) are added to these media in consideration of the cells to be induced, and the cell differentiation is carried out.

BHAWNA CHANDRAVANSHI ET AL; JOURNAL OF CELLULAR PHYSIOLOGY, vol. 233, no. 2, 11 August 2017 (2017-08-11), pages 1627-1637, describes reprogramming mouse embryo fibroblasts to functional islets without genetic manipulation.

D. FOMINA-YADLIN ET AL; PNAS, vol. 107, no. 34, 9 August 2010 (2010-08-09), pages 15099-15104, describes a high-content screening for small-molecule inducers of insulin expression.

The corresponding PhD Dissertation Fomina-Yadlin Dina A: ""Small molecule inducers of insulin expression in pancreatic alpha cells", 6 July 2011 (2011-07-06), pages 1-210, discloses i.a. that BRD7389 is a small-molecule activator of insulin expression in alpha cells and the effect of p53 activation by GW8510 on beta cell differentiation.

WO 2014/001771 A1 discloses a method for reprogramming a somatic cell (pancreatic ductal cell, a skin fibroblast or a keratinocyte) to a pancreatic beta-cell.

EP 3 031 905 A1 discloses the use of forskolin and/or CHIR99021 for generating pancreatic hormone-producing cells from progenitor cells.

### PRIOR ART DOCUMENTS

### PATENT DOCUMENT

Patent Document 1: WO 2018/062269

### NON-PATENT DOCUMENT

Non-Patent Document 1: Current Pathobiology Reports, 2015, Vol. 3, pp. 57-65
Non-Patent Document 2: Cell Stem Cell, 2014, Vol. 14, pp. 228-236
Non-Patent Document 3: Nature Communications, 2016, Vol. 7, p. 10080
Non-Patent Document 4: Journal of Biological Chemistry, 2017, Vol. 292, pp. 19122-19132

### SUMMARY OF THE INVENTION

### PROBLEM TO BE SOLVED BY THE INVENTION

One of the problems in differentiation induction of cells is that it is difficult to achieve a sufficient amount of differentiation induction to tolerate practical use, even if differentiation induction to a desired cell can be successful. This problem is also the same when an insulin-producing cell is induced.

The present invention is primarily directed to providing a new in vitro process capable of efficiently performing direct conversion or induction from a somatic cell to an insulin-producing cell by a low molecular weight compound without performing artificial gene transfer.

### MEANS FOR SOLVING THE PROBLEMS

As a result of intensive studies, the present inventors have found that in direct induction from a somatic cell to an insulin-producing cell, the above-mentioned problem can be solved by culturing a cell in the absence of fetal bovine serum (FBS) which is usually added to a culture medium, or by culturing a cell by adding insulin in a larger amount than usual to the culture medium, and the present invention was completed.

The present invention includes the following as claimed in appended claims 1 to 8:
[1.] An in vitro process for producing an insulin-producing cell by direct differentiation induction from a somatic cell, comprising a step of culturing a fibroblast or a mesenchymal stem cell in a differentiation induction medium containing at least 20 µg/mL of insulin, an RSK inhibitor, a GSK3 inhibitor and a cAMP inducer, wherein the RSK inhibitor is BRD7389 or BI-D1870, the GSK3 inhibitor is CHIR99021 and the cAMP inducer is forskolin, wherein the differentiation induction medium is serum-free.
[2.] The in vitro process for producing an insulin-producing cell according to [1.], wherein the differentiation induction medium further contains the PI3K inhibitor LY294002.
[3.] The in vitro process for producing an insulin-producing cell according to [1.] or [2.], wherein the differentiation inducing medium contains insulin the range of 80 µg/mL to 120 µg/mL.
[4.] The in vitro process for producing an insulin-producing cell according to any one of [1.] to [3.], wherein the somatic cell is a fibroblast.
[5.] A differentiation induction composition for producing an insulin-producing cell by directly inducing differentiation from a fibroblast or a mesenchymal stem cell, wherein the composition contains at least 20 µg/mL of insulin, an RSK inhibitor, a GSK3 inhibitor and a cAMP inducer, wherein the RSK inhibitor is BRD7389 or BI-D1870, the GSK3 inhibitor is CHIR99021 and the cAMP inducer is forskolin. wherein the composition is serum-free.
[6.] The differentiation induction composition for producing an insulin-producing cell according to [5.], further containing a PI3K inhibitor, wherein the PI3K inhibitor is LY294002.
[7.] The differentiation induction composition for producing an insulin-producing cell according to [5.] or [6.], comprising insulin the range of 80 µg/mL to 120 µg/mL.
[8.] The differentiation induction composition according to any one of [5.] to [7.], wherein the somatic cell is a fibroblast.

### EFFECT OF THE INVENTION

According to the present invention, direct conversion or induction from somatic cells to insulin-producing cells with high insulin secretion ability can be efficiently performed without performing artificial gene transfer. The insulin-producing cells obtained according to the present invention are useful in regenerative medicine and the like.

### BRIEF DESCRIPTION OF THE DRAWINGS

Figure 1 represents the amount of insulin secreted by insulin-producing cells directly differentiated from human fibroblasts, with or without fetal bovine serum (FBS). The vertical axis indicates insulin secretion per mg of total protein of the cell (µU/mg). In each experimental result, the left column and the right column, respectively, show the results obtained when fetal bovine serum (FBS) is contained, and the results obtained when fetal bovine serum (FBS) is not contained.
Figure 2 represents the amount of insulin secreted by insulin-producing cells directly differentiated from human fibroblasts under the presence of 20 µg/mL or 120 µg/mL of insulin. The vertical axis indicates insulin secretion per mg of total protein of the cell (µU/mg). In each experimental result, the left column and the right column, respectively, show the results obtained when 20 µg/mL of insulin was contained, and the results obtained when 120 µg/mL of insulin was contained.
Figure 3 represents the amount of insulin secreted by insulin-producing cells directly differentiated from human fibroblasts. The vertical axis indicates insulin secretion per mg of total protein of the cell (µU/mg).
Figure 4 represents the amount of insulin secreted by insulin-producing cells directly differentiated from human fibroblasts. The vertical axis indicates insulin secretion per mg of total protein of the cell (µU/mg).
Figure 5 represents the amount of insulin secreted by insulin-producing cells directly differentiated from human adipose tissue-derived mesenchymal stem cells (AdMSC). The vertical axis indicates insulin secretion per mg of total protein of the cell (µU/mg).

### EMBODIMENTS FOR CARRYING OUT THE PRESENT INVENTION

Hereinafter, the present invention will be described in detail.

### 1 Method of producing insulin-producing cells

The process for producing an insulin-producing cell according to the invention (hereinafter, referred to as "the present invention process") is a process for producing an insulin-producing cell by direct differentiation induction from a somatic cell, comprising a step of culturing a somatic cell in a serum-free differentiation induction medium. Moreover, it is a process for producing an insulin-producing cell by direct differentiation induction from a somatic cell, comprising a step of culturing a somatic cell in a differentiation induction medium containing 20 µg/mL or more of insulin.

As mentioned above, one of the gists of the present invention is to produce an insulin-producing cell by culturing a somatic cell in a serum-free differentiation induction medium substantially free of serum such as fetal bovine serum (FBS). Moreover, a gist of the present invention can include producing an insulin-producing cell by containing a relatively large amount, i.e., 20 µg/mL or more of insulin in a differentiation induction medium.

Here, "serum-free" refers to the state of being substantially free of unadjusted or unpurified serum, and in the present invention, even if a differentiation induction medium is contaminated with a purified component derived from blood or animal tissue, the medium is referred to as a serum-free differentiation induction medium, so long as it does not substantially contain an unadjusted or unpurified serum.

Thus, the present invention process comprises a step of culturing a somatic cell in a differentiation induction medium, which is serum-free and contains 20 µg/mL or more of insulin.

The above step is a step of culturing a somatic cell in the presence of an RSK inhibitor, a GSK3 inhibitor, and a cAMP inducer. The above step may be a step of culturing a somatic cell in the further presence of other inhibitors, inducers, and the like, if necessary, such as a PI3K inhibitor.

### 1.1 Somatic cells

Cells of an organism can be classified into somatic and germ cells. Any somatic cell can be used as a starting material in the present invention process. The somatic cell is not particularly limited, and may be either a primary cell taken from a living body or a cell from an established cell line. Somatic cells at various stages of differentiation, e.g., terminally differentiated somatic cells (e.g., fibroblasts, umbilical vein endothelial cells (HUVEC), liver cells (Hepatocytes), bile duct cells (Biliary cells), pancreatic alpha cells (Pancreatic α cells), pancreatic acinar cells (Acinar cells), pancreatic duct gland cells (Ductal cells), small intestinal crypt cells (Intestinal crypt cells), and the like), somatic cells on the way to terminal differentiation (e.g., mesenchymal stem cells, neural stem cells, endodermal progenitor cells, and the like), or somatic cells that have been reprogrammed and acquired pluripotency, can be used in the present invention process. The somatic cells that can be used in the present invention process include any somatic cells, for example, cells of the hematopoietic system (various lymphocytes, macrophages, dendritic cells, bone marrow cells, and the like), cells derived from organs (hepatocytes, splenocytes, pancreatic cells, kidney cells, lung cells, and the like), cells of the muscle tissue system (skeletal muscle cells, smooth muscle cells, myoblasts, cardiomyocytes, and the like), fibroblasts, neurons, osteoblasts, chondrocytes, endothelial cells, interstitial cells, adipocytes (white adipocytes, and the like), embryonic stem cells (ES-cells), and the like. Preferably, fibroblasts or mesenchymal stem cells can be used.

The fibroblasts that can be used in the present invention are not particularly limited, and can include, for example, dermal fibroblasts, adventitial fibroblasts, cardiac fibroblasts, pulmonary fibroblasts, uterine fibroblasts, and villous mesenchymal fibroblasts, which are major cell components forming connective tissues in various tissues and organs, and produce collagen fibers.

The mesenchymal stem cells that can be used in the present invention are not particularly limited, and can include, for example, adipose tissue-derived mesenchymal stem cells, bone marrow-derived mesenchymal stem cells, umbilical cord-derived mesenchymal stem cells, umbilical cord blood-derived mesenchymal stem cells, dental pulp-derived mesenchymal stem cells, placenta-derived mesenchymal stem cells, amniotic membrane-derived mesenchymal stem cells, endometrium-derived mesenchymal stem cells, synovium-derived mesenchymal stem cells, dermal tissue-derived mesenchymal stem cells, and periodontal ligament-derived mesenchymal stem cells.

Examples of the source of the above-mentioned somatic cells include, but are not limited to, humans, mammals other than humans, and animals other than mammals (birds, reptiles, amphibians, fish, and the like). As a source of somatic cells, humans and mammals other than humans are preferred, and humans are particularly preferred. When insulin-producing cells are produced by the present invention process for the purpose of administration to humans, preferably, somatic cells harvested from a donor having a matched or similar type of histocompatibility antigen to the recipient can be used. Somatic cells harvested from the recipient itself may be used for the production of insulin-producing cells.

### 1.2 Somatic cell culture

Culturing of somatic cells in the present invention process can be carried out by a conventional method, except that the differentiation induction medium does not contain serum such as fetal bovine serum (FBS), and contains 20 µg/mL or more of insulin. Furthermore, a medium, a temperature, and other conditions can be selected depending on the type of a somatic cell to be used, and culturing of a somatic cell can be carried out in the presence of various inhibitors described above (and, optionally, an inducer or an activator).

In the present invention process, by culturing a somatic cell in a differentiation induction medium containing various inhibitors and the like described above, an insulin-producing cell can be produced from a somatic cell by one step culture.

Moreover, by selecting a somatic cell for use which can be easily cultured, it is possible to increase the number of cells in advance and convert the somatic cell which has reached a substantially confluent state into an insulin-producing cell. Thus, a scaled-up production of insulin-producing cells is also easy.

The differentiation induction medium or the subculture medium for a somatic cell, which are the bases for carrying out the present invention, can be selected from known or commercially available media. For example, MEM (Minimum Essential Medium), DMEM (Dulbecco's Modified Eagle's Medium), or DMEM/F 12, which are a common medium, or a modified medium thereof, can be used by adding appropriate components (serum, proteins, amino acids, saccharides, vitamins, fatty acids, antibiotics, and the like) thereto.

The present invention process, the differentiation induction medium contains insulin in an amount of 20 µg/mL or more, or 25 µg/mL or more, and more preferably within the range of 80 µg/mL to 120 µg/mL.

When a certain amount of insulin is previously contained in the known or commercially available basal induction medium, insulin may be added thereto to adjust the insulin content to the above amount.

As a culture condition, a general condition of cell culture can be selected. Conditions of 37°C and 5% CO₂ and the like are exemplified. It is preferred to change the medium at appropriate intervals during culture (preferably once every one to five days, and more preferably once every two to four days). When the present invention process is carried out using fibroblasts as materials, insulin-producing cells appear in between six to eight days and 12 days under conditions of 37°C and 5% CO₂. When the present invention process is carried out using mesenchymal stem cells as materials, insulin-producing cells appear in about one week under the conditions of 37°C and 5% CO₂.

For culturing somatic cells, a cell culture container such as a plate, a dish, a cell culture flask, a cell culture bag, or the like can be used. Note that, as the cell culture bag, one having gas permeability is suitable. A large culture vessel may be used when large amounts of cells are required. The culture can be carried out in either an open system or a closed system, but when the purpose is administration or the like of the obtained insulin-producing cells to a human, it is preferable to carry out the culture in a closed system.

### 1.3 Inhibitors and the like according to the present invention

### 1.3.1 RSK inhibitors

RSK (Ribosomal S6 Protein Kinase) is widely expressed in cells and is one of the serine-threonine kinases that respond to various growth factors. It is classified into subfamilies with molecular weights of 70 kDa and 90 kDa. In particular, the 90 kDa RSK subfamily is activated by phosphorylation by ERK belonging to MAPK signaling pathway, and is derived from four genes in mammals. Activated 90 kDa RSK phosphorylate a variety of down-stream proteins, including Ribosomal protein S6, and regulate cell viability, cell proliferation, and differentiation in a variety of ways.

The term "in the presence of an RSK inhibitor" refers to a culture condition capable of inhibiting RSK, and the means thereof is not particularly limited, and substances that inhibit the activity of RSK, for example, RSK signal inhibiting means such as an anti-RSK antibody or an RSK inhibitor, can be used. Moreover, since RSK is activated when its own specific site is phosphorylated, the means of inhibiting the above-mentioned phosphorylation can also be used to inhibit the RSK signal.

For the RSK inhibitor, BRD7389 or BI-D1870 can be used.

BRD7389 (CAS No.: 376382-11-5)

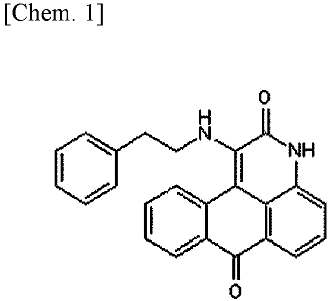

SL 0101-1 (CAS No.: 77307-50-7)*
BI-D1870 (CAS No.: 501437-28-1)
LJH685 (CAS No.: 1627710-50-2)*
LJI308 (CAS No.: 1627709-94-7)*
FMK (CAS No.: 821794-92-7)*
RMM46 (CAS No.: 1307896-46-3)*
CMK (CAS No.: 821794-90-5)*
Carnosol (CAS No.: 5957-80-2)*
Bix 02565 (CAS No.: 1311367-27-7)*
(* RSK inhibitors not included in the present invention)

The concentration of the RSK inhibitor varies depending on a somatic cell or the like to be used, but is not particularly limited, and may be appropriately determined, and can be used, for example, within the range of 0.05 µmol/L to 50 µmol/L, and preferably within the range of 0.1 µmol/L to 20 µmol/L.

### 1.3.2 GSK3 inhibitors

GSK3 (glycogen synthase kinase-3) was found as a protein kinase that phosphorylates and inactivates glycogen synthase. In mammals, GSK3 is classified into two isoforms: 51 kDa α (GSK3α) and 47 kDa β (GSK3β). GSK3 has the activity of phosphorylating various proteins and is involved not only in glycogen metabolism but also in physiological phenomena such as cell division and cell growth.

The term "in the presence of a GSK3 inhibitor" refers to a culture condition capable of inhibiting GSK3, and the means thereof is not particularly limited, and substances that inhibit the activity of GSK3, for example, GSK3 signal inhibiting means such as an anti-GSK3 antibody and a GSK3 inhibitor, can be used. Moreover, since GSK3 loses its activity when its own specific site is phosphorylated, the means of promoting the above-mentioned phosphorylation can also be used to inhibit the GSK3 signal.

For the GSK3 inhibitor, CHIR99021 is used.

CHIR99021 (CAS No.: 252917-06-9)

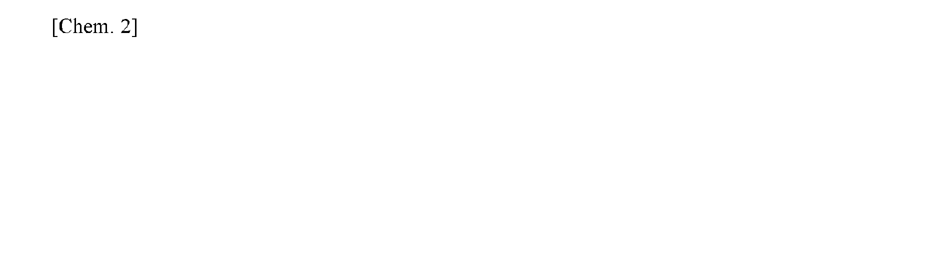

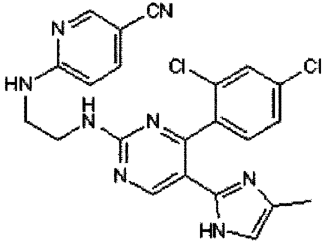

BIO ((2'Z,3'E)-6-Bromoindirubin-3'-oxime) (CAS No.: 667463-62-9)*
Kenpaullone (CAS No.: 142273-20-9)*
A1070722 (CAS No.: 1384424-80-9)*
SB216763 (CAS No.: 280744-09-4)*
CHIR98014 (CAS No.: 556813-39-9)*
TWS119 (CAS No.: 601514-19-6)*
Tideglusib (CAS No.: 865854-05-3)*
SB415286 (CAS No.: 264218-23-7)*
Bikinin (CAS No.: 188011-69-0)*
IM-12 (CAS No.: 1129669-05-1)*
1-Azakenpaullone (CAS No.: 676596-65-9)*
LY2090314 (CAS No.: 603288-22-8)*
AZD1080 (CAS No.: 612487-72-6)*
AZD2858 (CAS No.: 486424-20-8)*
AR-A014418 (CAS No.: 487021-52-3)*
TDZD-8 (CAS No.: 327036-89-5)*
Indirubin (CAS No.: 479-41-4)*
(* GSK3 inhibitor not included in the present invention)

The concentrations of the GSK3 inhibitor varies depending on a somatic cell and the like to be used, but is not particularly limited, and may be appropriately determined, and can be used, for example, within the range of 0.05 µmol/L to 20 µmol/L, and preferably within the range of 0.1 µmol/L to 10 µmol/L.

### 1.3.3 cAMP inducers

cAMP (cyclic adenosine monophosphate) is a second messenger that is involved in a variety of intracellular signaling events. cAMP is produced intracellularly by cyclization of adenosine triphosphate (ATP) by adenylyl cyclase.

The term "in the presence of a cAMP inducer" refers to a culture condition capable of inducing cAMP, and the means thereof is not particularly limited, and for example, any means capable of increasing the intracellular cAMP concentrations can be used. Substances that can induce cAMP by acting directly on adenylyl cyclase, an enzyme involved in the production of cAMP, substances that can promote the expression of adenylyl cyclase, and substances that inhibit phosphodiesterase, an enzyme that degrades cAMP, can be used as means to increase intracellular cAMP concentrations. It is also possible to use dibutyryl cAMP, a structural analogue of cAMP, which has the same effect as cAMP in cells.

For the cAMP inducer (adenylate cyclase activator) forskolin is used.

forskolin (CAS No.: 66428-89-5)

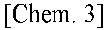

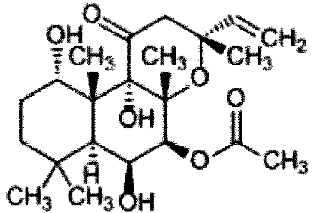

Isoproterenol (CAS No.: 7683-59-2)*
NKH477 (CAS No.: 138605-00-2)*
PACAP1-27 (CAS No.: 127317-03-7)*
PACAP1-38 (CAS No.: 137061-48-4)*
(* cAMP inducer not included in the present invention)

The concentration of the cAMP inducer varies depending on a somatic cell and the like to be used, but is not particularly limited, and may be appropriately determined, and can be used, for example, within the range of 0.2 µmol/L to 50 µmol/L, and preferably within the range of 1 µmol/L to 30 µmol/L.

### 1.3.4 PI3K inhibitor

PI3K (Phosphoinositide 3-kinase) is an enzyme that phosphorylates an inositol phospholipid, and a phosphoinositide produced activates PDK1. The PDK1 further phosphorylates AKT and activates PDK1/AKT signaling pathway. LY294002 is a selective inhibitor of PI3K and inhibits activation of PDK1/AKT signaling pathway by suppressing the production of phosphoinositide.

The term "in the presence of a PI3K inhibitor" refers to a culture condition capable of inhibiting PI3K, and the means thereof is not particularly limited, and any means capable of inhibiting PI3K can be used. In the present invention, substances that act directly on PI3K to inhibit its function (e.g., anti-PI3K antibodies, and other agents), agents that inhibit the production of PI3K themselves, and the like can be used. PI3K can also be inhibited by inhibiting the upstream signaling pathway of PI3K.

For the PI3K inhibitor, LY294002 is used.

LY294002 (CAS No.: 154447-36-6)

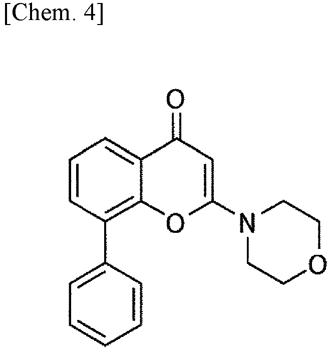

Buparlisib (CAS No.: 944396-07-0)*
TGR-1202 (CAS No.: 1532533-67-7)*
PI-103 (CAS No.: 371935-74-9)*
IC-87114 (CAS No.: 371242-69-2)*
Wortmannin (CAS No.: 19545-26-7)*
ZSTK474 (CAS No.: 475110-96-4)*
AS-605240 (CAS No.: 648450-29-7)*
PIK-90 (CAS No.: 677338-12-4)*
AZD6482 (CAS No.: 1173900-33-8)*
Duvelisib (CAS No.: 1201438-56-3)*
TG100-115 (CAS No.: 677297-51-7)*
CH5132799 (CAS No.: 1007207-67-1)*
CAY10505 (CAS No.: 1218777-13-9)*
PIK-293 (CAS No.: 900185-01-5)*
CZC24832 (CAS No.: 1159824-67-5)*
Pilaralisib (CAS No.: 934526-89-3)*
AZD8835 (CAS No.: 1620576-64-8)*
(* PI3K inhibitors not included in the present invention)

The concentration of the PI3K inhibitor varies depending on a somatic cell and the like to be used, but is not particularly limited, and may be appropriately determined, and can be used, for example, within the range of 0.1 µmol/L to 20 µmol/L, and preferably within the range of 0.5 µmol/L to 10 µmol/L.

### 1.4 Insulin-producing cells

By the present invention process described above, a cell population containing insulin-producing cells can be obtained. The insulin-producing cell produced by the present invention process is also within the scope of the present invention. The insulin-producing cell produced by the present invention process may be not only a terminally differentiated cell but also a progenitor cell destined to differentiate into an insulin-producing cell.

The insulin-producing cell produced by the present invention process is a so-called low molecular weight compound-induced insulin-producing cell (ciIPCs), which is induced to differentiate directly from a somatic cell by a low molecular weight compound, and is distinguished from that induced to differentiate by gene transfer.

The insulin-producing cell produced by the present invention process can be detected, identified, and isolated using, for example, a morphological change of a cell, a characteristic property of an insulin-producing cell, or a specific marker (e.g., an anti-insulin antibody). Moreover, the secretory capacity of the obtained insulin-producing cell can also be evaluated by quantifying the amount of secreted insulin using sandwich ELISA.

Quarantine methods (detection with an antibody) can be used to detect specific markers, and as for protein molecules, detection may be performed with quantitation of their mRNA quantities. An antibody that recognizes the specific marker of the insulin-producing cell is also useful for isolating and purifying the insulin-producing cell obtained by the present invention process.

The insulin-producing cell produced by the present invention process can be used, for example, for methods of tissue repair, improvement of blood insulin concentration, and the like. By transplanting the insulin-producing cell produced by the present invention process, a pharmaceutical composition for tissue repair or the like can be produced. Pancreas transplantation or islet transplantation has become a radical treatment for patients with type 1 diabetes, who are congenitally almost completely deficient in insulin secretion, to alleviate and cure their symptoms. Moreover, the number of patients with type 2 diabetes is expected to increase further in Japan and overseas, and cause medical costs to rise, but transplantation of pancreatic β-cells secreting insulin may be an effective therapy. As a means for treating pancreatic diseases such as diabetes, a process for producing insulin-producing cells and a method for transplanting insulin-producing cells have been developed. For example, transplantation of insulin-producing cells under the kidney capsule or transplantation into the liver via the portal vein is expected to be used for the treatment of severe pancreatic diseases such as diabetes.

When the insulin-producing cell produced by the present invention process is used as a pharmaceutical composition, it may be prepared in a formulation suitable for administration to an individual, such as by mixing the insulin-producing cell with a pharmaceutically acceptable carrier, in a conventional method. Examples of the carrier can include physiological saline and distilled water for injection which is made isotonic by adding glucose or other adjuvants (e.g., D-sorbitol, D-mannitol, sodium chloride, and the like). Furthermore, buffering agents (e.g., a phosphate buffer, a sodium acetate buffer), painless agents (e.g., benzalkonium chloride, procaine hydrochloride, and the like), stabilizers (e.g., human serum albumin, polyethylene glycol, and the like), preservatives, antioxidants, and the like may be blended.

The insulin-producing cell produced by the present invention process may also be made into a composition further combined with other cells or components effective for improving the functional performance and the engraftment of the insulin-producing cell.

Furthermore, the insulin-producing cell produced by the present invention process can also be used for screening of a pharmaceutical candidate compound which acts on an insulin-producing cell and for evaluating the safety of a pharmaceutical candidate compound. Insulin-producing cells are an important tool for evaluating the toxicity of a pharmaceutical candidate compound. According to the present invention process, a large number of insulin-producing cells can be acquired in a single operation, and then it is possible to obtain reproducible research results without being affected by a lot difference in cells.

### 2 Compositions

The differentiation induction composition according to the present invention (hereinafter, referred to as "the present invention composition") is a composition for producing an insulin-producing cell by directly inducing differentiation from a somatic cell, wherein the composition is serum-free and contains 20 µg/mL or more of insulin.

As described above, one of the gists of the present invention is that serum such as fetal bovine serum (FBS) is not substantially contained in the composition for producing an insulin-producing cell by directly inducing differentiation from a somatic cell, and that a relatively large amount, i.e., 20 µg/mL or more of insulin is contained in the composition for producing an insulin-producing cell by direct differentiation induction from a somatic cell.

Thus, in the above, the present invention composition is serum-free and contains 20 µg/mL or more of insulin.

Moreover, the composition of the present invention comprises an RSK inhibitor, a GSK3 inhibitor and a cAMP inducer, as defined above. Furthermore, the present invention composition may optionally comprise other inhibitors, inducers, and the like such as a PI3K inhibitor, as required as defined above.

Specific inhibitors and the like may have two or more types of inhibitory effects, and in this case, the presence of one may be deemed to be the presence of a plurality of inhibitors and the like. Specific examples and preferred examples of the above-mentioned inhibitors and inducers and the like are the same as those described above.

The present invention composition can be used as a medium for producing insulin-producing cells from somatic cells.

As the present invention composition, a medium not containing serum such as fetal bovine serum (FBS) and/or containing 20 µg/mL or more of insulin in a basal medium produced by mixing components necessary for culturing cells can be exemplified. Further exemplified is a medium containing an RSK inhibitor as an active ingredient, and further, if necessary, a GSK3 inhibitor, and/or a cAMP inducer or a PI3K inhibitor in the basal medium. The active ingredient described above may be contained in a concentration effective for producing an insulin-producing cell, and the concentration may be appropriately determined by those skilled in the art. The basal medium may be selected from known or commercially available media. For example, MEM (Minimum Essential Medium), DMEM (Dulbecco's Modified Eagle's Medium), DMEM/F12, or RPMI1640, which are a common medium, or a medium modified therewith, can be used as the basal medium.

The present invention composition, contains insulin in an amount of 20 µg/mL or more, or 25 µg/mL or more, and more preferably within the range of 80 µg/mL to 120 µg/mL.

When a certain amount of insulin is previously contained in the known or commercially available differentiation induction medium, the present invention composition can be prepared by adding insulin so that insulin is contained in the above amount.

A known medium component described above herein, such as serum, proteins (albumin, transferrin, a growth factor, and the like), amino acids, saccharides, vitamins, fatty acids, antibiotics, and the like may be further added to the medium according to the present invention composition.

Substances effective for inducing differentiation into insulin-producing cells described above may further be added to the medium according to the present invention composition.

### EXAMPLES

Hereinafter, the present invention will be illustrated in more detail with reference to Examples, but the present invention is not limited to the scope of the Examples.

### EXAMPLE 1: Production of insulin-producing cells

### - Direct induction from a human fibroblast to an insulin-producing cell -

### (1) Human fibroblasts

The human fibroblasts used as the material were purchased from DS Pharma Biomedical Co., Ltd. The fibroblasts are derived from 38-year-old human skin.

### (2) Direct induction from human fibroblasts into insulin-producing cells

Human fibroblasts were seeded in 35-mm dishes coated with gelatin (Cat#: 190-15805, manufactured by Wako Pure Chemical Industries, Ltd.) at 5 × 10⁴ cells/dish, and cultured in DMEM medium (manufactured by Gibco) supplemented with 10% fetal bovine serum (FBS), 100 U/mL penicillin, and 100 µg/mL streptomycin until confluent under conditions of 37°C and 5% CO₂. Note that DMEM refers to Dulbecco's Modified Eagle's Medium.

The medium of dishes of the human fibroblasts described above was replaced with the differentiation induction medium described below.
- Differentiation induction medium: Advanced DMEM/F-12 (Cat#: 12634010; manufactured by Gibco) supplemented with ITS-X (Cat#: 51500056, manufactured by Gibco), non-essential amino acids (NEAA; Cat#: 11140050, manufactured by Gibco), Glutamine (manufactured by Gibco; final concentration: 2 mmol/L), Nicotinamide (Cat#: 72340-100G, manufactured by Sigma-Aldrich; final concentration: 10 mmol/L), Exendin-4 (Cat#: av120214, manufactured by Abcam; final concentration: 100 ng/mL), 100 U/mL penicillin, 100 µg/mL streptomycin, insulin, and low molecular weight compounds described below, with or without addition of 10% fetal bovine serum (FBS, manufactured by Hyclone).

Subsequently, the cells were cultured under conditions of 37°C and 5% CO₂ while the medium was changed to the medium of the same composition every three days.

Human recombinant insulin (Cat#: 093-06351; Wako) was added to the differentiation induction medium as required so that the medium contained 20 to 120 µg/mL of insulin.

### <Low Molecular Weight Compounds>

3 µM CHIR99021 (Cat#: 13122, Cayman Chemical)
7.5 µM forskolin (Cat#: 063-02193, Wako)
1.5 µM BRD7389 (Cat#: ab146161, Abcam)
5 µM LY294002 (Cat#: 70920, Cayman Chemical)

### (3) Results

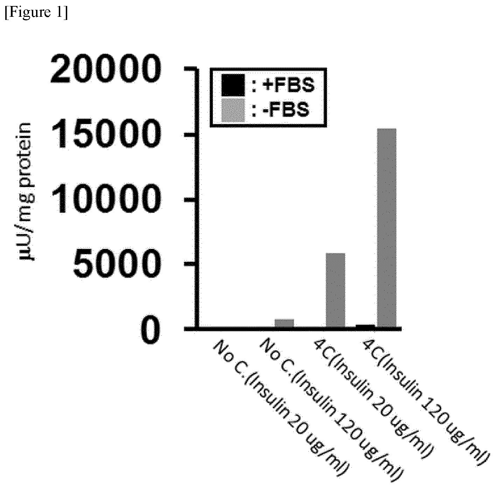

The results of culturing for 14 days according to (2) described above are shown in Figures 1 to 4.

In the Figures, the term "4C" refers to the above-mentioned four low molecular weight compounds, and the term "3C" refers to three compounds of the above-mentioned four low molecular weight compounds, which are CHIR99021, BRD7389, and forskolin. The term "+FBS" indicates the presence of FBS in the medium, and the term "-FBS" indicates the absence of FBS in the medium. Moreover, the term "No C" or the term "No Compound" indicates the absence in the medium of the above-mentioned four low molecular weight compounds (4C) or 3C. Thus, for example, the term "No C. -FBS" represents experimental results obtained when no 4C is present and no FBS is present, and the term "4C-FBS" represents experimental results obtained when 4C is present and no FBS is present.

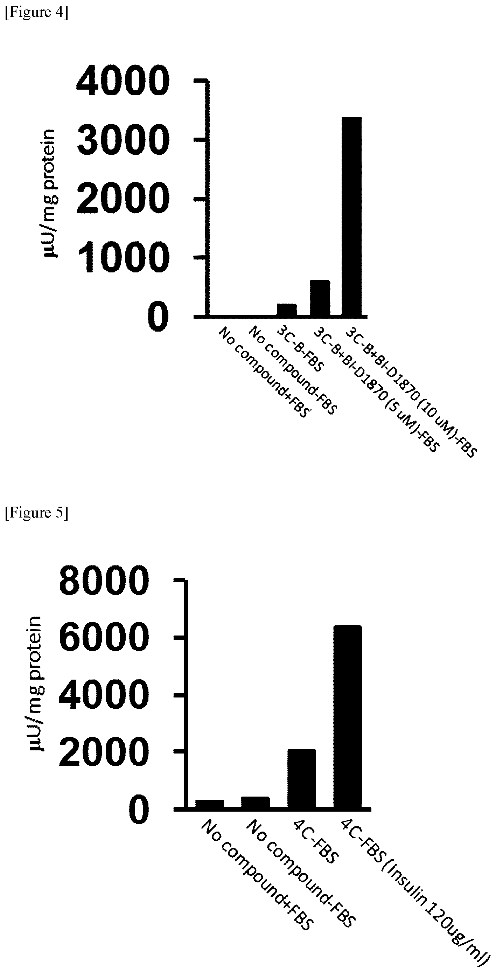

In Fig. 4, the term "B" refers to BRD7389, and the term "3C-B" represents the experimental results obtained in the absence of BRD7389 in 3C, namely, with two factors of CH and F. The term "3C-B+BI-D1870" represents the experimental results obtained when BRD7389 was absent among 3C, and instead BI-D1870 (Cat#: 15264, Cayman Chemical), an RSK inhibitor, was present at a final concentration of 5 µM or 10 µM.

As shown in Fig. 1, by induction without fetal bovine serum (FBS) in the differentiation induction medium, the amount of insulin secretion increased by about 20 to 50 times, and insulin-producing cells with a higher secretory capacity were efficiently induced directly from human fibroblasts.

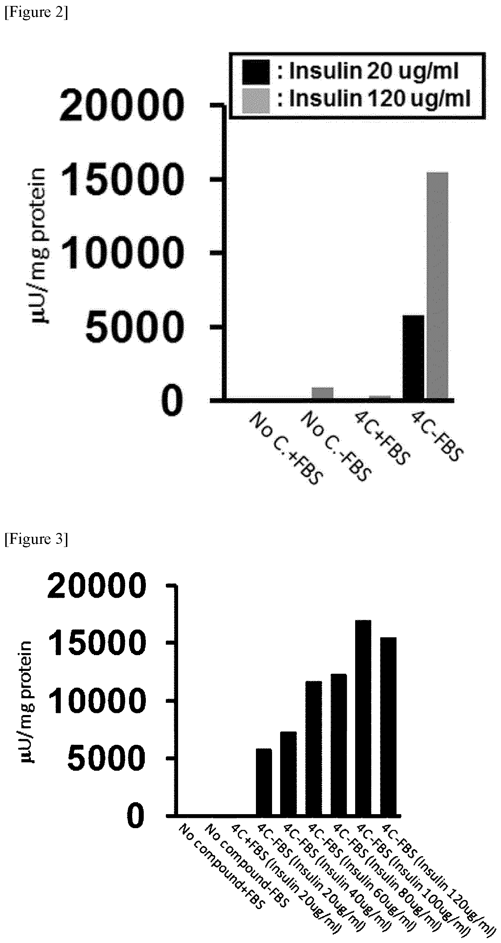

As shown in Fig. 2, by induction with a high concentration of insulin (120 µg/mL) in the differentiation induction medium, the amount of insulin secretion increased by about 3 times, and insulin-producing cells with a higher secretory capacity were efficiently induced directly from human fibroblasts.

From the above-mentioned, it is considered that the effects of FBS and high-concentration insulin on insulin secretion increase are independent of each other.

As shown in Fig. 3, the higher the concentration of insulin was in the differentiation induction medium up to 100 µg/mL, the greater was the amount of insulin secreted. Thus, insulin-producing cells with a high secretory capacity were obtained in an insulin concentration-dependent manner directly from human fibroblasts.

From the results of Fig. 4, the two factors, in the absence of LY294002, and further in the absence of the RSK inhibitor (BRD7389) among the three factors consisting of the RSK inhibitor (BRD7389), the GSK inhibitor (CHIR99021), and the cAMP inducer (forskolin), led to a low level of insulin secretion. It is suggested that the presence of RSK inhibitors is important to the induction to the insulin-producing cells. On the other hand, insulin-producing cells with a higher secretory capacity were also obtained by three factors in which BI-D1870, another RSK inhibitor, was present instead of BRD7389.

### EXAMPLE 2: Production of insulin-producing cells

### - Direct Induction from Human Mesenchymal Stem Cells to Insulin-Producing Cells -

### (1) Human mesenchymal stem cells

Human mesenchymal stem cells isolated from adipose tissue were purchased from Takara Bio Co., Ltd.

### (2) Direct Induction into Insulin-Producing Cells from Human Mesenchymal Stem Cells

Human mesenchymal stem cells were seeded in 35-mm dishes coated with gelatin (Cat#: 190-15805, manufactured by Wako Pure Chemical Industries, Ltd.) at 5 × 10⁴ cells/dish, and cultured in Mesenchymal Stem Cell Growth Medium 2 (Cat#: C-28009; manufactured by Takara Bio Co., Ltd.) supplemented with 100 U/mL penicillin and 100 µg/mL streptomycin until the cells were almost confluent under conditions of 37°C and 5% CO₂.

The medium of dishes of the human mesenchymal stem cells described above was replaced with the differentiation induction medium described below.
- Differentiation induction medium: Advanced DMEM/F-12 (Cat#: 12634010; manufactured by Gibco) supplemented with ITS-X (Cat#: 51500056, manufactured by Gibco), non-essential amino acids (NEAA; Cat#: 11140050, manufactured by Gibco), Glutamine (manufactured by Gibco; final concentration: 2 mmol/L), Nicotinamide (Cat#: 72340-100G, manufactured by Sigma-Aldrich; final concentration: 5 mmol/L), Exendin-4 (Cat#: av120214, manufactured by Abcam; final concentration: 50 ng/mL), 100 U/mL penicillin, 100 µg/mL streptomycin, and low molecular weight compounds described below, with or without addition of 10% fetal bovine serum (FBS, manufactured by Hyclone).

Subsequently, the cells were cultured under conditions of 37°C and 5% CO₂ while the medium was changed to the medium of the same composition every three days.

Note that in experiments in which the induction medium should contain 120 µg/mL of insulin, human recombinant insulin (Cat#: 093-06351; Wako) was added to adjust the medium to contain 120 µg/mL of insulin.

### <Low Molecular Weight Compounds>

0.5 µM CHIR99021 (Cat#: 13122, Cayman Chemical)
3.75 µM forskolin (Cat#: 063-02193, Wako)
0.2 µM BRD7389 (Cat#: ab146161, Abcam)
2.5 µM LY294002 (Cat#: 70920, Cayman Chemical)

### (3) Results

The results of culturing for 14 days according to the (2) described above are shown in Fig. 5.

In the Figure, the term "4C" refers to the above-mentioned four low molecular weight compounds. The term "+FBS" indicates the presence of FBS in the medium, and the term "-FBS" indicates the absence of FBS in the medium. Also, the term "No compound" indicates the absence of the above-mentioned four low molecular weight compounds in the medium. Thus, for example, the term "No Compound-FBS" represents experimental results obtained when no 4C is present and no FBS is present, and the term "4C-FBS" represents experimental results obtained when 4C is present and no FBS is present.

As shown in Fig. 5, also in direct differentiation induction from a human adipose tissue-derived mesenchymal stem cell (AdMSC), insulin secretion was sufficiently confirmed even when fetal bovine serum (FBS) was not contained in the differentiation induction medium. With the induction by containing a high concentration (120 µg/mL) of insulin in the differentiation induction medium, the amount of insulin secreted increased markedly, and insulin-producing cells with a higher secretory capacity were efficiently induced directly from human mesenchymal stem cells.

## Claims

1. An in vitro process for producing an insulin-producing cell by direct differentiation induction from a somatic cell, comprising a step of culturing a fibroblast or a mesenchymal stem cell in a differentiation induction medium containing at least 20 µg/mL of insulin, an RSK inhibitor, a GSK3 inhibitor and a cAMP inducer, wherein the RSK inhibitor is BRD7389 or BI-D1870, the GSK3 inhibitor is CHIR99021 and the cAMP inducer is forskolin, wherein the differentiation induction medium is serum-free.

2. The in vitro process for producing an insulin-producing cell according to claim 1, wherein the differentiation induction medium further contains the PI3Kinhibitor LY294002.

3. The in vitro process for producing an insulin-producing cell according to claim 1 or 2, wherein the differentiation inducing medium contains insulin the range of 80 µg/mL to 120 µg/mL.

4. The in vitro process for producing an insulin-producing cell according to any one of Claims 1 to 3, wherein the somatic cell is a fibroblast.

5. A differentiation induction composition for producing an insulin-producing cell by directly inducing differentiation from a fibroblast or a mesenchymal stem cell, wherein the composition contains at least 20 µg/mL of insulin, an RSK inhibitor, a GSK3 inhibitor and a cAMP inducer, wherein the RSK inhibitor is BRD7389 or BI-D1870, the GSK3 inhibitor is CHIR99021 and the cAMP inducer is forskolin. wherein the composition is serum-free.

6. The differentiation induction composition for producing an insulin-producing cell according to claim 5, further containing a PI3K inhibitor, wherein the PI3Kinhibitor is LY294002.

7. The differentiation induction composition for producing an insulin-producing cell according to claim 5 or 6, comprising insulin the range of 80 µg/mL to 120 µg/mL.

8. The differentiation induction composition according to any one of Claims 5 to 7, wherein the somatic cell is a fibroblast.

## Patentansprüche

1. *In* vitro-Verfahren zur Herstellung einer Insulin-produzierenden Zelle durch direkte Differenzierungsinduktion aus einer somatischen Zelle, umfassend einen Schritt des Kultivierens eines Fibroblasten oder einer mesenchymalen Stammzelle in einem Differenzierungsinduktionsmedium, das mindestens 20 µg/ml Insulin, einen RSK-Inhibitor, einen GSK3-Inhibitor und einen cAMP-Induktor enthält, wobei der RSK-Inhibitor BRD7389 oder BI-D1870 ist, der GSK3-Inhibitor CHIR99021 ist und der cAMP-Induktor Forskolin ist, wobei das Differenzierungsinduktionsmedium serumfrei ist.

2. *In* vitro-Verfahren zur Herstellung einer Insulin-produzierenden Zelle nach Anspruch 1, wobei das Differenzierungsinduktionsmedium des Weiteren den PI3K-Inhibitor LY294002 enthält.

3. *In* vitro-Verfahren zur Herstellung einer Insulin-produzierenden Zelle nach Anspruch 1 oder 2, wobei das Differenzierungsinduktionsmedium Insulin im Bereich von 80 µg/ml bis 120 µg/ml enthält.

4. *In* vitro-Verfahren zur Herstellung einer Insulin-produzierenden Zelle nach einem der Ansprüche 1 bis 3, wobei die somatische Zelle ein Fibroblast ist.

5. Differenzierungsinduktionszusammensetzung zur Herstellung einer Insulin-produzierenden Zelle durch direktes Induzieren der Differenzierung aus einem Fibroblasten oder einer mesenchymalen Stammzelle, wobei die Zusammensetzung mindestens 20 µg/ml Insulin, einen RSK-Inhibitor, einen GSK3-Inhibitor und einen cAMP-Induktor enthält, wobei der RSK-Inhibitor BRD7389 oder BI-D1870 ist, der GSK3-Inhibitor CHIR99021 ist und der cAMP-Induktor Forskolin ist, wobei die Zusammensetzung serumfrei ist.

6. Differenzierungsinduktionszusammensetzung zur Herstellung einer Insulin-produzierenden Zelle nach Anspruch 5, des Weiteren enthaltend einen PI3K-Inhibitor, wobei der PI3K-Inhibitor LY294002 ist.

7. Differenzierungsinduktionszusammensetzung zur Herstellung einer Insulin-produzierenden Zelle nach Anspruch 5 oder 6, umfassend Insulin im Bereich von 80 µg/ml bis 120 µg/ml.

8. Differenzierungsinduktionszusammensetzung nach einem der Ansprüche 5 bis 7, wobei die somatische Zelle ein Fibroblast ist.

## Revendications

1. Procédé in vitro pour la production d'une cellules produisant de l'insuline par induction de différenciation directe à partir d'une cellule somatique, comprenant une étape de culture d'un fibroblaste ou d'une cellule souche mésenchymateuse dans un milieu d'induction de différenciation contenant au moins 20 µg/ml d'insuline, un inhibiteur de RSK, un inhibiteur de GSK3 et un inducteur de cAMP, dans lequel l'inhibiteur de RSK est BRD7389 ou BI-D1870, l'inhibiteur de GSK3 est CHIR99021 et l'inducteur de cAMP est la forskoline, dans lequel le milieu d'induction de différenciation est exempt de sérum.

2. Procédé in vitro de production d'une cellule produisant de l'insuline selon la revendication 1, dans lequel le milieu d'induction de différenciation contient en outre l'inhibiteur de PI3K LY294002.

3. Procédé in vitro de production d'une cellule produisant de l'insuline selon la revendication 1 ou 2, dans lequel le milieu induisant la différenciation contient de l'insuline dans la plage de 80 µg/ml à 120 µg/ml.

4. Procédé in vitro de production d'une cellule produisant de l'insuline selon l'une quelconque des revendications 1 à 3, dans lequel la cellule somatique est un fibroblaste.

5. Composition d'induction de différenciation pour produire une cellule produisant de l'insuline en induisant directement une différenciation à partir d'un fibroblaste ou d'une cellule souche mésenchymateuse, dans laquelle la composition contient au moins 20 µg/ml d'insuline, un inhibiteur de RSK, un inhibiteur de GSK3 et un inducteur de cAMP, dans laquelle l'inhibiteur de RSK est BRD7389 ou BI-D1870, l'inhibiteur de GSK3 est CHIR99021 et l'inducteur de cAMP est la forskoline, dans laquelle la composition est exempte de sérum.

6. Composition d'induction de différenciation pour produire une cellule produisant de l'insuline selon la revendication 5, contenant en outre un inhibiteur de PI3K, dans laquelle l'inhibiteur de PI3K est LY294002.

7. Composition d'induction de différenciation pour produire une cellule produisant de l'insuline selon la revendication 5 ou 6, comprenant de l'insuline dans la plage de 80 µg/ml à 120 µg/ml.

8. Composition d'induction de différenciation selon l'une quelconque des revendications 5 à 7, dans laquelle la cellule somatique est un fibroblaste.
